# EUROPEAN PATENT APPLICATION

(11) **EP 1 938 840 A1**
(43) Date of publication of application: **02.07.2008**
(21) Application number: 06127212.6
(22) Date of filing: 27.12.2006
(51) Int. Cl.: A61K 45/06, A61K 31/381, A61K 9/20, A61K 9/48

(54) **Duloxetine composition**

(71) Applicant: LEK Pharmaceuticals d.d., 1526 Ljubljana (SI)
(72) Inventor: The designation of the inventor has not yet been filed
(74) Representative: Demsar, Marija

(57) **Abstract**

The present invention relates to a stable pharmaceutical composition comprising duloxetine or a pharmaceutically acceptable salt thereof and a method for making such composition. In particular, the composition comprises duloxetine hydrochloride and a separating layer comprising a water soluble inorganic salt.

## Description

### Field of the invention

The present invention relates to a stable pharmaceutical composition comprising duloxetine or a pharmaceutically acceptable salt thereof and a method for making such composition.

### Background of the invention

Duloxetine hydrochloride is a selective serotonin and norepinephrine reuptake inhibitor (SSNRI). Its chemical designation is (+)-(*S*)-*N*-methyl-γ-(1-naphthyloxy)-2-thiophenepropylamine hydrochloride. Duloxetine hydrochloride is administered orally and used to treat major depressive disorder, stress urinary incontinence and diabetic peripheral neuropathic pain. Duloxetine hydrochloride is a white to slightly brownish white solid, which is slightly soluble in water and commercially available as Cymbalta® and Yentreve®.

The chemical structure is

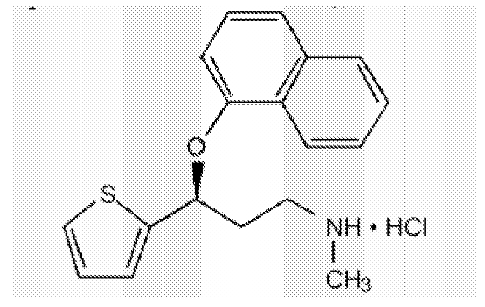

Due to its chemical structure, duloxetine is prone to acid-catalyzed hydrolysis, which results in the formation of potentially harmful degradation products and a decrease in the biovailability of duloxetine. Duloxetine is unstable in solution at pH values less than 2.5, therefore it is degraded in the gastric environment of the stomach. Acid hydrolysis of its ether linkage results in thienyl alcohol and 1-naphthol. Hydrolysis is not desirable as 1-naphthol has toxic properties. Therefore, it is advisable to use enteric coated dosage forms, which pass through the stomach unchanged and do not release the active substance until they have reached the small intestine. Enteric coatings have been used for many years to arrest the release of the drug from orally ingestible dosage forms. Depending upon the composition and/or thickness, the enteric coatings are resistant to stomach acid for required periods of time before they begin to disintegrate and permit slow release of the drug in the lower part of stomach or upper part of the small intestine. A pellet form comprising duloxetine has been proposed as the most suitable form of administration for consistent plasma concentration profiles.

However, the use of gastric polymers in the enteric formulation gives rise to several problems regarding stability of duloxetine. Since conventionally used gastric coatings contain acidic functional groups, they can cause acid-catalysed degradation of duloxetine. Also, it has been shown that duloxetine reacts with some of these coatings to form amide adducts. These interactions reduce the potency of the drug and can alter the physical characteristics of the coating.

Therefore, it has been proposed that a separating or barrier layer can be used between the duloxetine-containing layer and the enteric coating to prevent the contact between the duloxetine-containing layer and the polymers in the enteric coating. This helps to achieve the desired level of stability of duloxetine in the gastric pellets.

US patent 5,508,276 relates to a duloxetine enteric formulation in the form of enteric pellets wherein the enteric layer comprises hydroxypropylmethylcellulose acetate succinate (HPMCAS). The enteric duloxetine pellet comprises a) a core consisting of duloxetine and a pharmaceutically acceptable excipient; b) an optional separating layer; c) an enteric layer comprising HPMCAS and a pharmaceutically acceptable excipient; d) an optional finishing layer. In a preferred embodiment, the optional separating layer comprises sucrose to improve acid resistance and stability. In US 5,508,276 it is described that duloxetine reacts with many enteric coatings to form slowly or even insoluble coating, which can lead to the decreased bioavailability of duloxetine. Based on the compatibility with duloxetine, hydroxypropylmethylcellulose acetate succinate was chosen as a enteric polymer which is compatible with the desired release of duloxetine from the pharmaceutical formulation. HPMCAS is partially neutralized with ammonium ions to the degree that from about 0% to about 25% of the succinic acid groups are neutralized.
US patent application 20060165776 relates to enteric compositions suitable for oral administration comprising duloxetine or its pharmaceutical derivatives and methods for preparing such compositions. Such compositions contain a core consisting of a duloxetine or its pharmaceutical derivatives, the said core comprised of a pharmaceutically inert nuclei and the duloxetine or its pharmaceutical derivatives thereof compressed together, an intermediate and an enteric layer. The composition is free of alkaline reacting compounds.

The present invention provides an improved stable pharmaceutical composition comprising duloxetine or a pharmaceutically acceptable salt thereof suitable for oral administration.

### Summary of invention

The present invention employs a water soluble solid in a separating layer which can be interposed between a core and an enteric coat. The water soluble solid such as sodium chloride occurs as crystals or other leachable form. In this way the separating layer can provide a barrier during storage between a core containing duloxetine or a pharmaceutically acceptable salt thereof, such as duloxetine hydrochloride, and an enteric coat, and which is readily lost upon administration and penetration of the enteric coat.

In a first aspect, the invention relates to a pharmaceutical composition comprising a core comprising duloxetine or a pharmaceutically acceptable salt thereof and a separating layer comprising an inorganic salt.

In a second aspect, the invention relates to a method for preparing such a pharmaceutical composition, the method comprising the following steps: forming a core containing duloxetine or a pharmaceutically acceptable salt thereof and applying a separating layer comprising a water soluble inorganic salt to the core.

### Detailed description of the invention

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Duloxetine is an active substance, which is known to be unstable in acidic media. It is also incompatible with several common pellet ingredients, such as lactose. It is formulated into an enteric pellet to protect it from the acidic gastric milieu and allow timely dissolution of the drug once the pellets have reached the jejunum. As the polymers conventionally used for enteric coating contain acidic functional groups, the coating must be separated from the duloxetine-containing layer of the pharmaceutical composition in order to avoid the interactions.

There are problems in finding compatible materials. Table 1 below shows the degradation of duloxetine in 1:1 binary mixtures with 20% (w/w) of water added, after 14 days at 50°C.

| **Excipient** | **Sum of degradation products** |
|---|---|
| None | 0.50 % |
| Microcrystalline cellulose | 1.51 % |
| Sucrose | 0.81 % |
| Cornstarch | 1.07 % |
| Hydroxypropylmethylcellulose (HPMC) | 0.83 % |
| Lactose | 25.61 % |
| Lactose, anhydrous | 14.30 % |
| Talc | 0.68 % |
| Sodium Chloride | 0.72 % |
| HPMC acetate succinate (HPMCAS) | 93.89 % |
| Eudragit L | 70.31 % |
| Polyvinylacetate phthalate (Sureteric) | 30.79 % |
| (Sureteric is a fully formulated ready to use mixture of excipients: polyvinyl acetate phthalate, talc, polyethylene glycol, titanium dioxide, sodium bicarbonate, triethyl citrate, purified stearic acid, sodium alginate, and colloidal silicon dioxide) | |

The present invention uses a water soluble salt, typically an inorganic salt such as sodium chloride, to form an efficient separating layer of a pharmaceutical formulation containing duloxetine or a pharmaceutically acceptable salt thereof. A film-forming polymer, such as hydroxypropylmethylcellulose (HPMC), may optionally be included in the separating layer. Thus, in one aspect, the salt-containing layer comprises a polymer matrix with embedded salt crystals.

The inorganic salt may be dissolved in a coating liquid together with a polymer and sprayed onto the duloxetine-containing core of the formulation. Upon drying, the evaporation of the solvent causes the salt to crystallize and form a substantially uniform layer of crystals embedded in the structure of the polymer film. This structure provides an efficient physical barrier preventing the interactions between duloxetine and the components of the gastric/enteric coating. Crystals form an efficient barrier since their highly organized internal structure prevents diffusion of any species. The polymeric film surrounding the crystals is still permeable for different species, although its decreased content also dramatically decreases permeability of the insulating layer. Polymer film can be used to bind the crystals together.

Moreover, the use of a water-soluble salt component in the separating layer according to the invention allows the separating layer to dissolve rapidly, for example once water has penetrated across the enteric layer, thus minimizing the impact that this layer could have on the release of the active ingredient duloxetine or a pharmaceutically acceptable salt thereof from the pellet. In this way, the separating layer allows an efficient stabilization of the active compound without interfering with its release from the dosage form.

Duloxetine may be present in the form of a pharmaceutically acceptable salt of duloxetine, typically duloxetine hydrochloride.

The water soluble inorganic salt is preferably an inorganic salt formed between a mineral acid and an alkali metal or alkaline earth metal. Salts according to the invention include but are not limited to sodium phosphate, dibasic sodium bicarbonate, sodium carbonate, sodium sulfate, potassium chloride, potassium phosphate, dibasic potassium bicarbonate, potassium carbonate, magnesium chloride, sodium chloreide and calcium chloride. Chlorides are preferred, but other halides are candidates. Sodium chloride is the most preferred salt. Thus, the salt may be a binary salt.

The separating layer forms a barrier, intermediate or insulating layer which is applied to the core which comprises the active ingredient duloxetine.

An enteric coat or layer relates to a coat comprised of a polymer or other material which prevents premature disintegration of the pharmaceutical in the acidic environment of the stomach and promotes rapid release of the drug in the intestine. The enteric coat is external of the separating layer.
Ratios given herein are %w/w unless the context clearly requires otherwise.

### Duloxetine core

The preferred pharmaceutical composition of the present invention employs duloxetine or a pharmaceutically acceptable salt thereof, such as duloxetine hydrochloride, sprayed onto neutral cores. A suitable polymeric binder may be added. The polymer binder can be chosen among hydrophilic or hydrophobic polymers such as derivatives of cellulose (for example methylcellulose, hydroxypropyl cellulose, hypromellose, ethylcellulose); polyvinylpirolidone (for example povidone, crospovidone, copovidone); polymethacrylates (for example Eudragit RS, RL); and various other substances such as for example hydroxypropyl starch, gelatine and other water soluble polymers.

Additionally, other substances known in the art to facilitate processing may be added, for example talc, colloidal silicon dioxide or magnesium silicate as antitacking agents.

A preferred core is a neutral inert bead which acts as a pharmaceutically acceptable carrier. The inert bead may be prepared from a pharmaceutically acceptable carrier such as starch, sucrose, microcrystalline cellulose, vegetable gum or other suitable pharmaceutically acceptable material.

Typically duloxetine or a pharmaceutically acceptable salt thereof will be present in the pharmaceutical composition of the present invention in an amount within the range of 5 to 50% of the product, preferably 15 to 30%. However, the amount of duloxetine or a pharmaceutically acceptable salt thereof depends on the desired dose for administration.

A polymer may be added as a binder, typically in a 1:20 to 2:1 ratio (polymer : active). Suitable polymers include hydroxypropylmethylcellulose, hydroxypropylcellulose and polyvinylpyrrolidone.

The size of the core may be about 0.1 mm to about 2mm in diameter.

The core containing duloxetine or a pharmaceutically acceptable salt thereof may be produced using techniques commonly used in the art, such as suspension/solution layering, powder layering, rotor peletizing or an extrusion/spheronisation process.

### Separating layer

According to the present invention, the preferred separating layer comprises small dispersed crystals of a water soluble inorganic salt bound together with either solid bridges of partially dissolved crystalline substance or by a polymer dissolved or dispersed in water. Thus, the separating layer comprises a water soluble inorganic salt which is present in the form of crystals.

A suitable procedure for applying the separating layer to the core is powder layering using rotor processor with powder feeder and a spraying nozzle. With this method, crystals of the inorganic salt are added in powder form and the amount of binder minimised.

In a preferred embodiment, the separating layer consists substantially of crystals connected via solid bridges of partially solubilised crystals and does not include a polymeric binder. Thus, it is possible to obtain a separating layer without the addition of a polymer binder by spraying the core spheres with water and adding crystals which are connected via liquid bridges. Upon evaporation of water, solid bridges are formed from partially dissolved crystals. With this method of coating, water partially dissolves the salt in crystalline form. The saturated solution is sticky and binds non-dissolved particles together. The coating process is preferably carried out to continuously add powder and water and dry the product at the same time. Binding and drying occur simultaneously. The final product does not contain water since this is removed during process. Without wishing to be bound by theory, it is believed that In the final product, non-dissolved particles are bound with solid bridges which re-crystallised from saturated solution.

When using this method, the particle size of the crystals is important. To obtain a smooth surface, a particle size of about 10 microns or less is preferred. Therefore, in a preferred embodiment, the particle size of the inorganic salt crystal is about 10 microns or less.

Thus, in another embodiment, the separate layer includes inorganic salt in the form of crystals and a polymer. Thus, the salt crystals are embedded in a polymer matrix. In preferred embodiments, the percentage of crystals present in the polymer matrix is 5 to 100%, more preferably 5 to 50%. Effective polymer to crystal ratio according to the embodiments of the invention is thus preferably between 0:1 and 20:1.

Polymers for use in the separating layer may be selected from various pharmaceutically acceptable polymers, such as polyvinylpyrrolidone, hypromellose, hydroxypropyl cellulose, hydroxypropylmethylcellulose or neutral polymethacrylates.

A suitable method for applying the separating layer comprising an inorganic salt in the form of crystals which are embedded in a polymer is by coating in bottom spray fluid bed equipment (Wuerster column) using a solution or dispersion of inorganic salt crystals. This method requires a polymeric binder which can be either dissolved or dispersed in the coating media. Additionally, the separating layer can contain substances for ease of processing, for example a filler such as talc or other excipients, such as silicon dioxide, colorants, pigments or other suitable substances known in the art. Crystals of the inorganic salt are formed during drying of the solution droplets which are applied onto the core. Rapid drying of solution droplets ensures a small crystal size and their easy integration in polymer matrix.

An alternative method for producing crystal containing is coating with dispersion of water-soluble crystals in non aqueous media such as for instance ethanol. When coating with dispersion of crystals is employed particle size of crystals is preferably 10 microns or less.

Alternative processes for applying the separating layer include spray coating in a perforated drum, top spray fluid bed coater and other processes known in the art.

### Enteric layer

In one embodiment, the formulation comprises an enteric layer which is applied to the separating layer. The enteric/gastric coat or layer can be applied using one of the spray coating techniques: perforated drum, bottom spray fluid bed coater (Wuerster column), top spray fluid bed coater or rotor processor. During research work it was concluded that polymethacrylates are not appropriate polymers for duloxetine formulation since they have a tendency to form complexes with duloxetine, this results in inadequate dissolution of duloxetine hydrochloride from pellets.

A known polymer used in gastric coating for duloxetine-containing pellets is HPMCAS. The inventors have demonstrated that also polymers such as polyvinyl acetate phthalate (PVAP) are appropriate for gastric coating. Pellets produced with PVAP are stable due to the effective insulating layer and at the same time provide protection from acidic media as demonstrated in dissolution data of Example 2 (see below). Therefore, the enteric layer according to the invention may comprise PVAP or HPMCAS.

The pharmaceutical formulation according the invention is for oral administration and is preferably in the form of a pellet, preferably an enteric pellet.

In another aspect, the invention relates to a method for preparing a pharmaceutical composition, the method comprising the following steps: forming a core containing duloxetine or a pharmaceutically acceptable salt thereof and applying a separating layer comprising a water soluble inorganic salt to the core.

Additionally, an enteric layer as described herein may be added. In the separating layer, the salt is preferably present in the form of salt crystals. The inorganic salt may be in the form of crystals in a solution or dispersion applied to the core. Alternatively, crystal formation can occur upon application to the core. Optionally, the separating layer may comprise a polymer as defined herein.

The invention will be further understood with references to the non-limiting examples.

### Examples

| Example 1 | mg |
|---|---|
| Sugar Spheres 30/35 | 60,189 |

| API LAYER | |
|---|---|
| Duloxetine Hydrochloride | 67,358 |
| Hypromellose | 22,453 |
| INSULATING LAYER | |
| Hypromellose | 16,020 |
| Sodium Chloride | 16,020 |
| Talc | 8,010 |

| ENTERIC LAYER | |
|---|---|
| Poly(methacrylic acid, ethyl acrylate) 1 : 1 (30% water dispersion) Eudragit L 30 D-55 | 148,200 |
| Triethylcitrate | 13,338 |
| Talc | 22,230 |

Hypromellose is dissolved in water, duloxetine is dispersed water using Ultraturax and then mixed with the hypromellose solution. The resulting dispersion has 15% of dry matter. The dispersion is applied onto sugar spheres in Wurster column (Glatt, GCGP 1). During dispersion, coating product temperature is held constant at 40° C. The product is then dried.

To make the separating layer, sodium chloride and hypromellose are dissolved in water, talc is added and dispersed using Ultraturax. The resulting dispersion has 10 % of dry matter.

The dispersion is applied onto drug loaded spheres in Wurster column. During coating product temperature is held constant at 40 °C. The product is then dried.

To make the enteric layer, triethylcitrate is dissolved in water, talc is added and dispersed. The resulting dispersion is added to Eudragit L 30 D 55 dispersion and the two are mixed. The final dispersion has 20% of dry matter. The dispersion is applied onto the core spheres prepared above and coated with a separating layer in a Wurster column. During coating product temperature is held constant at 30 ° C. The product is then dried.

All three coatings are applied sequentially. The batch size of finished product is 1,2 kg.

Pellets are filled into hard gelatin capsules (size No. 1).

| Example 2 | mg |
|---|---|
| Sugar Spheres 30/35 | 120,000 |

| API LAYER | |
|---|---|
| Duloxetine Hydrochloride | 67,358 |
| Hypromellose | 32,642 |
| Talc | 10,000 |
| INSULATING LAYER | |
| Hypromellose | 8,000 |
| Mannitol | 12,000 |
| Talc | 20,000 |

| ENTERIC LAYER | |
|---|---|
| Polyvinyl acetate phthalate Sureteric (comerically available ready to use gastric coat) | 130,000 |

Preparation of Example 2 is essentially the same as in Example 1. Sureteric is dispersed in water to obtain coating dispersion. The dispersion has 15% of dry matter. The dispersion is applied onto spheres with a separating layer in a Wurster column. During dispersion, coating product temperature is held constant at 30 °C. The product is then dried.

| Example 3 | mg |
|---|---|
| Sugar Spheres 30/35 | 60,189 |

| API LAYER | |
|---|---|
| Duloxetine Hydrochloride | 67,358 |
| Hypromellose | 22,453 |
| INSULATING LAYER | |
| Hypromellose | 8,000 |
| Mannitol | 12,000 |
| Talc | 20,000 |

| ENTERIC LAYER | |
|---|---|
| Poly(methacrylic acid, ethyl acrylate) 1 : 1 (30% water dispersion) | |
| Eudragit L 30 D-55 | 148,200 |
| Triethylcitrate | 13,338 |
| Talc | 22,230 |

Preparation of Example 3 is essentially the same as in Example 1.

| Example 4 | mg |
|---|---|
| Sugar Spheres 30/35 | 60,189 |

| API LAYER | |
|---|---|
| Duloxetine Hydrochloride | 67,358 |
| Hypromellose | 22,453 |

| INSULATING LAYER | |
|---|---|
| Hypromellose | 16,020 |
| Sodium Chloride | 16,020 |
| Talc | 8,010 |

| ENTERIC LAYER | |
|---|---|
| Polyvinyl acetate phthalate | |
| Sureteric (comerically available ready to use gastric coat) | 130,000 |

Preparation of Example 4 is essentially the same as in Example 1. Sureteric is dispersed in water to obtain coating dispersion. The dispersion has 15% of dry matter. The dispersion is applied onto spheres with insulating layer in a Wurster column. During dispersion, coating product temperature is held constant at 30 °C. The product is then dried.

Samples made as shown in Examples 1 and 3 were used to assess the impact of sodium chloride, which is included in the separating layer, on the stability of duloxetine in the formulations. The results of stress stability testing of these samples, shown in Table 1, clearly demonstrate that the inclusion of sodium chloride into the insulating layer significantly improves the stability of duloxetine. At a highly stressful condition (50°C, 100% relative humidity), the extent of hydrolysis is much more limited and the formation of the toxic degradation product 1-naphthol, is much less significant, when sodium chloride is used.

**Table 2: Degradation products of duloxetine in the pellets, after 7 days at 50°C / 100 % relative humidity.**

| Degradation product | Example 1 (with NaCl) | Example 3 (without NaCl) |
|---|---|---|
| 1-naphthol | 0.74 | 5.01 |
| 4-naphthyl derivate | 0.17 | 2.31 |
| Max other | 0.12 | 1.20 |
| **Sum** | **1.12** | **8.67** |

After oral administration of a pharmaceutical formulations it is essential that the active pharmaceutical ingredient incorporated in the formulation is completely released from the formulation in the gastrointestinal tract, enabling that the total amount of the active pharmaceutical ingredient is available for the absorption from the gastrointestinal tract in the systemic circulation.

Duloxetine is an acid labile drug therefore it must be protected against acid hydrolysis in the stomach by the enteric coating formed by an enteric polymer. Enteric polymers are insoluble in acid environment in the stomach and soluble in the neutral conditions in the small intestine, thus they prevent the release of active pharmaceutical ingredient in the stomach and enable the release of active pharmaceutical ingredient in the small intestine, which is the major site for drug absorption.

We have unexpectedly observed that the complete release of duloxetine from the enteric coated pharmaceutical formulation can also be obtained by using polyvinyl acetate phthalate (Sureteric) as a enteric polymer.

The release of the active pharmaceutical ingredient from the pharmaceutical formulation is regularly tested in USP dissolution apparatus. The release of duloxetine from the pharmaceutical formulation was tested in USP Apparatus 1 (rotating basket) at 37°C and 100 rpm with 2 hours in 0.1 M HCl (gastric challenge) followed by 90 minutes in 900 ml of phosphate buffer (50 mM, pH 6.8). After two hours in 0.1 M HCl no significant amount of duloxetine was released from all of the tested formulations.

**Table 3.**

| | % of released duloxetine | | |
|---|---|---|---|
| Time in phosphate buffer (min) | Cymbalta 60 mg | Example 1 | Example 2 |
| 90 | 95,0 | 52,9 | 95,6 |

It is evident from Table 3 that practically complete (more than 90 %) amount of duloxetine is released from the reference formulation (Cymbalta®) containing hydroxypropylmethylcellulose acetate succinate as an enteric polymer and from Example 2 containing polyvinyl acetate phthalate (Sureteric) as an enteric polymer. On the other hand, only 53 % of duloxetine was released from Example 1 containing Eudragit L as an enteric polymer. This result indicates incompatibility between duloxetine hydrochloride and Eudragit L when both of them are in solution.

## Claims

1. A pharmaceutical composition comprising a core comprising duloxetine or a pharmaceutically acceptable salt thereof and a separating layer comprising a water soluble inorganic salt.

2. A pharmaceutical composition according to claim 1 further comprising an enteric layer.

3. A pharmaceutical composition according to claim 1 or claim 2 wherein the salt is a halide.

4. A pharmaceutical composition according to claim 3 wherein the halide is sodium chloride.

5. A pharmaceutical composition according to a preceding claim wherein the salt is present in the form of crystals.

6. A pharmaceutical composition according to a preceding claim wherein the particle size of the crystals is 10 microns or less.

7. A pharmaceutical composition according to a preceding claim wherein the separating layer further comprises a polymer.

8. A pharmaceutical composition according to claim 7 wherein the salt is present in the form of crystals embedded in a polymer matrix.

9. A pharmaceutical composition according to claim 7 or 8 wherein the polymer is polyvinylpyrrolidone, hypromellose, hydroxypropyl cellulose, hydroxypropylmethylcellulose or neutral polymethacrylates.

10. A pharmaceutical composition according to a preceding claim wherein the ratio of polymer to inorganic salt crystal is between 0:1 to 20:1.

11. A pharmaceutical composition according to claims 2 to 10 wherein the enteric layer comprises a polymer.

12. A pharmaceutical composition according to claim 11 wherein the polymer is PVAP.

13. A pharmaceutical composition according to a preceding claim comprising duloxetine hydrochloride.

14. A pharmaceutical composition according to a preceding claim wherein the composition is formulated as an enteric pellet.

15. A method for preparing a pharmaceutical composition as defined in a preceding claim, the method comprising the following steps: forming a core containing duloxetine or a pharmaceutically acceptable salt thereof and applying a separating layer comprising a water soluble inorganic salt to the core.

16. A method according to claim 15 wherein the organic salt is present in the form of crystals embedded in a polymer matrix.

17. The method of claim 15 or claim 16 further comprising applying an enteric layer to the separating layer.
